# EUROPEAN PATENT SPECIFICATION

(11) **EP 4 104 875 B1**
(45) Date of publication and mention of the grant of the patent: **01.11.2023**
(21) Application number: 21893116.0
(22) Date of filing: 30.07.2021
(51) Int. Cl.: A61L 28/00, A61K 36/232, A61K 36/30, A61K 36/185

(54) **CORROSION-RESISTANT STOMA LEAKAGE-PROOF PASTE CAPABLE OF ACHIEVING SELF-REGULATION OF STRENGTH AND PREPARATION METHOD THEREFOR**
KORROSIONSBESTÄNDIGE STOMA-LECKSICHERE PASTE ZUR ERZIELUNG EINER SELBSTREGULIERUNG DER FESTIGKEIT UND HERSTELLUNGSVERFAHREN DAFÜR
PÂTE ANTI-FUITE POUR STOMIE, RÉSISTANTE À LA CORROSION, CAPABLE DE RÉALISER UNE AUTORÉGULATION DE LA RÉSISTANCE ET PROCÉDÉ DE PRÉPARATION DE CELLE-CI

(30) Priority: 27.04.2021 CN 202110459156
(43) Date of publication of application: 21.12.2022
(73) Proprietor: ZHENDE MEDICAL CO., LTD., Shaoxing, Zhejiang 312035 (CN)
(72) Inventor: FENG, Lanqi, Shaoxing Zhejiang 312035 (CN); LU, Jianguo, Shaoxing Zhejiang 312035 (CN)
(74) Representative: Loo, Chi Ching
(86) International application number: PCT/CN2021/109804
(87) International publication number: WO 2022/227321

(56) References cited:
- CN-A- 106 728 727
- CN-A- 108 096 628
- CN-A- 108 744 066
- CN-A- 108 992 713
- CN-A- 109 456 544
- EA-A1- 201 500 032
- EA-B1- 025 271
- GB-A- 258 146
- TW-A- 200 831 117
- US-B2- 6 509 391

## Description

### TECHNICAL FIELD

The disclosure relates to a field of medical materials, in particular to a corrosion-resistant and leak-proof stoma paste with self-regulation of strength and a preparation method thereof.

### BACKGROUND ART

Currently, best-selling leak-proof pastes in China are mainly divided into alcoholic ones and non-alcoholic ones. The alcoholic paste present good shaping ability and short curing time, but it contains isopropanol and other ingredients, which can irritate skin to a certain extent and is not conducive to use of dermatitis patients. The non-alcoholic paste has no irritation to skin with poor shaping ability, and it is easy to be squeezed out when it is used with a stoma rubber tray, which increases difficulty of stoma care. A non-alcoholic leak-proof paste for stoma care and preparation method thereof are disclosed in Chinese patent application title by "A non-alcoholic leak-proof paste for stoma care and preparation method thereof (with an application publication number: CN108744066A)". A corrosion-resistant and leak-proof stoma paste and preparation method thereof are disclosed Chinese patent application document titled by "A corrosion-resistant and leak-proof stoma paste and preparation method thereof (with an application publication number: CN108992713A)". Inorganic clay is introduced into these two types of leak-proof pastes to improve corrosion-resistant performance of the pastes, with low paste strength.

US 6 509 391 discloses a mouldable mass for use with an ostomy appliance. The mass comprises 1-20 wt% of a styrene block copolymer with molecular weight of 20,000-15,000, 5-60 wt% of a tackifying liquid constituent, preferably polyisobutylene, 1-10 wt% of a waxy constituent and 20-70 wt% of a hydrocolloid, e.g. carrageenan. The mass may further comprise liquid paraffin in an amount of up to 30 wt%, and a filler such as talc, calcium carbonate or zinc oxide in an amount of 3-20 wt%, as well as agents for wound care. A type of paste is required in the market that can solve problems of poor corrosion resistance and low paste strength of existing leak-proof pastes.

Currently, the leak-proof paste only plays a simple role in filling gaps around the stoma and can only prevent generation of skin complications, but does not have a function of treating the skin complications. A type of paste is required in the market that can prevent leakage and treat the skin complications at the same time.

The disclosure solves above two problems, and provides a leak-proof paste with good corrosion resistance and high paste strength, which can treat the skin complications at the same time.

### SUMMARY

In order to solve shortcomings of the prior art, the present disclosure aims to provide a corrosion-resistant and leak-proof stoma paste with self-regulation of strength and preparation method thereof. A product of the present disclosure can self-regulate hardness of the paste according to amount of stoma excreta to improve corrosion resistance of the paste, and introduce stoma skin complication medicine, which can prevent leakage and play a therapeutic effect on the skin complications of the stoma at the same time.

In order to achieve above objects, the present invention is defined by the independent claims, while the dependent claims concern the preferred embodiments. According to the claimed invention a corrosion-resistant and leak-proof stoma paste with self-regulation of strength is provided, with a formula comprising 4-40 parts of liquid rubber with a molecular weight ranging from 2000 to 5000, 8-18 parts of mineral oil, 14-34 parts of iota-carrageenan, 10-20 parts of plant polysaccharide and 15-20 parts of sericite, by weight.

For the corrosion-resistant and leak-proof stoma paste with self-regulation of strength, the formula further includes 5-15 parts of the stoma skin complication medicine by weight.

For the corrosion-resistant and leak-proof stoma paste with self-regulation of strength, the stoma skin complication medicine includes 1-3 parts of Lithospermum Root, 1-3 parts of Angelica sinensis and 3-9 parts of sesame oil, by weight.

For the corrosion-resistant and leak-proof stoma paste with self-regulation of strength, a weight ratio of the Lithospermum Root to the sesame oil is 1: 3.

For the corrosion-resistant and leak-proof stoma paste with self-regulation of strength, the liquid rubber with the molecular weight ranging from 2000 to 5000 is one or more of polyisobutylene, liquid polyisoprene and liquid styrene-butadiene rubber with a molecular weight ranging from 2000 to 5000.

For the corrosion-resistant and leak-proof stoma paste with self-regulation of strength, the mineral oil is one or more of naphthenic oil, white oil and paraffin oil.

For the corrosion-resistant and leak-proof stoma paste with self-regulation of strength, the plant polysaccharide is one or more of tamarind seed polysaccharide, seaweed polysaccharide, chitosan oligosaccharide, tremella polysaccharide, hawthorn polysaccharide and oat β - glucan.

The present invention also provides a preparation method of a corrosion-resistant and leak-proof stoma paste with self-regulation of strength including the following steps:
In Step 1, materials prepared according to a formula.

The formula includes 4-40 parts of liquid rubber with a molecular weight ranging from 2000 to 5000, 8-18 parts of mineral oil, 14-34 parts of iota-carrageenan, 10-20 parts of plant polysaccharide and 15-20 parts of sericite, by weight.

In Step 2, the liquid rubber with the molecular weight ranging from 2000 to 5000, the mineral oil, the iota-carrageenan, the plant polysaccharide and the sericite are evenly mixed and stirred to obtain a mixed paste, which is packaged to obtain a finished product.

The present invention further provides a preparation method of a corrosion-resistant and leak-proof stoma paste with self-regulation of strength including the following steps:
In Step 1, materials are prepared according to a formula.

The formula includes 4-40 parts of liquid rubber with a molecular weight ranging from 2000 to 5000, 8-18 parts of mineral oil, 14-34 parts of iota-carrageenan, 10-20 parts of plant polysaccharide and 15-20 parts of sericite, 1-3 parts of Lithospermum Root, 1-3 parts of Angelica sinensis and 3-9 parts of sesame oil, by weight.

In Step 2, the sesame oil is heated to 90°C and then the weighed Lithospermum Root and the Angelica sinensis are added, decocted for 1h, and filtered to obtain medicinal oil.

In Step 3, the obtained medicinal oil, the liquid rubber with the molecular weight ranging from 2000 to 5000, the mineral oil, the iota-carrageenan, the plant polysaccharide and the sericite are evenly mixed and stirred to obtain a mixed paste, which is packaged to obtain a finished product.

According to the preparation method, a double planetary stirrer is adopted for stirring, with a rotating speed of 80-180 r/min.

The disclosure has advantages as follows.

In the disclosure, the iota-carrageenan is adopted, which can form a strong gel network structure with calcium ions in the stoma excreta. The gel network structure is elastic and insoluble in water and its strength increases with increase of the calcium ions in solution, so the paste can self-regulate its strength with amount of the stoma excreta.

In the disclosure, the sericite is adopted, which has unique volume expansion effect and a two-dimensional sheet configuration with a high aspect ratio, excellent reinforcement effect is exhibited in rubber products, so it can strengthen the paste jointly with the carrageenan.

In the disclosure, it is found that the iota-carrageenan and the sericite are cooperated to present a synergistic effect on the strength and corrosion resistance of the paste, thus improve the strength of the paste and enhance the corrosion resistance.

The Lithospermum Root is adopted in the stoma skin complication medicine. The Lithospermum Root contains shikonin, which has the good effect of eliminating putridity and engendering flesh. At the same time, it has been found in research that the Lithospermum Root can inhibit growth activity of Candida albicans, and protect and promote healing of wounds at the same time. The Angelica sinensis can promote blood circulation, nourish blood and promote granulation. The sesame oil has efficacy of detoxifying and promoting granulation. Combination of these three medicines presents remarkable astringent effect in treating skin diseases, so it can achieve astringent effect for skin complications and prevent sores.

### DETAILED DESCRIPTION

The disclosure will be described in the following in detail with reference to specific embodiments.

A corrosion-resistant and leak-proof stoma paste with self-regulation of strength is provided, with a formula comprising 4-40 parts of liquid rubber with a molecular weight ranging from 2000 to 5000, 8-18 parts of mineral oil, 14-34 parts of iota-carrageenan, 10-20 parts of plant polysaccharide and 15-20 parts of sericite, by weight. As an example, the liquid rubber with the molecular weight ranging from 2000 to 5000 is one or more of polyisobutylene, liquid polyisoprene and liquid styrene-butadiene rubber with a molecular weight ranging from 2000 to 5000. The mineral oil is one or more of naphthenic oil, white oil and paraffin oil. The plant polysaccharide is one or more of tamarind seed polysaccharide, seaweed polysaccharide, chitosan oligosaccharide, tremella polysaccharide, hawthorn polysaccharide and oat β - glucan. It should be note that all of examples herein are not exhaustive, and those that can achieve corresponding functions and effects are inspired by the present disclosure and are within protection scope of the present disclosure.

In order to achieve astringent effect for skin complications and prevent sores, the formula further includes 5-15 parts of the stoma skin complication medicine by weight. The stoma skin complication medicine includes 1-3 parts of Lithospermum Root, 1-3 parts of Angelica sinensis and 3-9 parts of sesame oil, by weight. As a preferred alternative, a weight ratio of the Lithospermum Root to the sesame oil is 1: 3. The Lithospermum Root contains shikonin, which has good effect of eliminating putridity and engendering flesh. At the same time, it has been found in research that the Lithospermum Root can inhibit growth activity of Candida albicans, and protect and promote healing of wounds at the same time. The Angelica sinensis can promote blood circulation, nourish blood and promote granulation. The sesame oil has efficacy of detoxifying and promoting granulation. Combination of these three medicines presents remarkable astringent effect in treating skin diseases, so it can achieve astringent effect for skin complications and prevent sores.

As an embodiment, a preparation method of a leak-proof paste without skin complication medicine of a stoma includes following steps.

In Step 1, materials prepared according to a formula.

In Step 2, a liquid rubber with a molecular weight ranging from 2000 to 5000, mineral oil, iota-carrageenan, plant polysaccharide and sericite are evenly mixed and stirred to obtain a mixed paste, which is packaged to obtain a finished product.

As another embodiment, a preparation method of a leak-proof paste with skin complication medicine of a stoma includes following steps.

In Step 1, materials are prepared according to a formula.

The formula includes 4-40 parts of liquid rubber with a molecular weight ranging from 2000 to 5000, 8-18 parts of mineral oil, 14-34 parts of iota-carrageenan, 10-20 parts of plant polysaccharide and 15-20 parts of sericite, 1-3 parts of Lithospermum Root, 1-3 parts of Angelica sinensis and 3-9 parts of sesame oil, by weight.

In Step 2, the sesame oil is heated to 90°C and then the weighed Lithospermum Root and the Angelica sinensis are added, decocted for 1h, and filtered to obtain medicinal oil.

In Step 3, the obtained medicinal oil, the liquid rubber with the molecular weight ranging from 2000 to 5000, the mineral oil, the iota-carrageenan, the plant polysaccharide and the sericite are evenly mixed, then put into and stirred in a double planetary stirrer with a rotating speed of 80-180 r/min, and a mixed paste is finally introduced into an automatic filling machine to finish filling. It should be noted that stirring and packaging are only for preferred embodiments, which is not limited and exhaustive. Whatever can realize functions of stirring and packaging is within the protection scope of the present disclosure.

Samples were obtained according to the formula and preparation method of following embodiments for Experiments 1-2.

### Embodiment 1

A novel corrosion-resistant and leak-proof stoma paste with self-regulation of strength for treating stoma skin complications according to an embodiment of the disclosure includes following raw materials in parts by weight: 34g of liquid rubber with a molecular weight ranging from 2000 to 5000, 10g of mineral oil, 15g of iota-carrageenan, 15g of plant polysaccharide, 20g of sericite powder, 2g of Lithospermum Root, 2g of Angelica sinensis and 6g of sesame oil.

The Lithospermum Root, the Angelica sinensis and the sesame oil are accurately weighed according to a formula; the sesame oil is heated to 90°C and then the weighed Lithospermum Root and the Angelica sinensis are added, decocted for 1h, and filtered to obtain medicinal oil; and the obtained medicinal oil, the liquid rubber with the molecular weight ranging from 2000 to 5000, the mineral oil, the iota-carrageenan, the plant polysaccharide and the sericite are evenly mixed in a mixed ratio, then put into and stirred in a double planetary stirrer, and then a mixed paste is introduced into an automatic filling machine to finish filling.

### Embodiment 2

A novel corrosion-resistant and leak-proof stoma paste with self-regulation of strength for treating stoma skin complications according to an embodiment of the disclosure includes following raw materials in g: 24g of liquid rubber with a molecular weight ranging from 2000 to 5000, 12g of mineral oil, 20g of iota-carrageenan, 20g of plant polysaccharide, 15g of sericite powder, 1g of Lithospermum Root, 1g of Angelica sinensis and 3g of sesame oil.

The Lithospermum Root, the Angelica sinensis and the sesame oil are accurately weighed according to a formula; the sesame oil is heated to 90°C and then the weighed Lithospermum Root and the Angelica sinensis are added, decocted for 1h, and filtered to obtain medicinal oil; and the obtained medicinal oil, the liquid rubber with the molecular weight ranging from 2000 to 5000, the mineral oil, the iota-carrageenan, the plant polysaccharide and the sericite are evenly mixed in a mixed ratio, then put into and stirred in a double planetary stirrer, and then a mixed paste is introduced into an automatic filling machine to finish filling.

### Embodiment 3

A novel corrosion-resistant and leak-proof stoma paste with self-regulation of strength for treating stoma skin complications according to an embodiment of the disclosure includes following raw materials in g: 16g of liquid rubber with a molecular weight ranging from 2000 to 5000, 15g of mineral oil, 28g of iota-carrageenan, 12g of plant polysaccharide, 18g of sericite powder, 3g of Lithospermum Root, 3g of Angelica sinensis and 9g of sesame oil.

The Lithospermum Root, the Angelica sinensis and the sesame oil are accurately weighed according to a formula; the sesame oil is heated to 90°C and then the weighed Lithospermum Root and the Angelica sinensis are added, decocted for 1h, and filtered to obtain medicinal oil; and the obtained medicinal oil, the liquid rubber with the molecular weight ranging from 2000 to 5000, the mineral oil, the iota-carrageenan, the plant polysaccharide and the sericite are evenly mixed in a mixed ratio, then put into and stirred in a double planetary stirrer, and then a mixed paste is introduced into an automatic filling machine to finish filling.

### Comparative Embodiment 1

A formula is as follows: 24g of liquid rubber with a molecular weight ranging from 2000 to 5000, 12g of mineral oil, 20g of plant polysaccharide, 15g of sericite powder, 1g of Lithospermum Root, 1g of Angelica sinensis and 3g of sesame oil.

The Lithospermum Root, the Angelica sinensis and the sesame oil are accurately weighed according to the formula; the sesame oil is heated to 90°C and then the weighed Lithospermum Root and the Angelica sinensis are added, decocted for 1h, and filtered to obtain medicinal oil; and the obtained medicinal oil, the liquid rubber with the molecular weight ranging from 2000 to 5000, the mineral oil, the plant polysaccharide and the sericite are evenly mixed in a mixed ratio, then put into and stirred in a double planetary stirrer, and then a mixed paste is introduced into an automatic filling machine to finish filling.

The formula and preparation steps are the same as those of Embodiment 2, except that iota-carrageenan is not added in the formula.

### Comparative Embodiment 2

A formula is as follows: 24g of liquid rubber with a molecular weight ranging from 2000 to 5000, 12g of mineral oil, 20g of iota-carrageenan, 20g of plant polysaccharide, 1g of Lithospermum Root, 1g of Angelica sinensis and 3g of sesame oil.

The Lithospermum Root, the Angelica sinensis and the sesame oil are accurately weighed according to a formula; the sesame oil is heated to 90°C and then the weighed Lithospermum Root and the Angelica sinensis are added, decocted for 1h, and filtered to obtain medicinal oil; and the obtained medicinal oil, the liquid rubber with the molecular weight ranging from 2000 to 5000, the mineral oil, the iota-carrageenan and the plant polysaccharide are evenly mixed in a mixed ratio, then put into and stirred in a double planetary stirrer, and then a mixed paste is introduced into an automatic filling machine to finish filling.

The formula and preparation steps are the same as those of Embodiment 2, except that sericite powder is not added in the formula.

### Comparative Embodiment 3

A formula is as follows: 24 g of liquid rubber with a molecular weight ranging from 2000 to 5000, 12 g of mineral oil, 20 g of iota-carrageenan, 20 g of plant polysaccharide, and 15 g of sericite powder.

The liquid rubber with the molecular weight ranging from 2000 to 5000, the mineral oil, the iota-carrageenan, the plant polysaccharide and the sericite are evenly mixed in a mixed ratio, then put into and stirred in a double planetary stirrer, and then a mixed paste is introduced into an automatic filling machine to finish filling.

The formula and preparation steps are the same as those of Embodiment 2, except that Lithospermum Root, Angelica sinensis and sesame oil are not added in the formula.

### Comparative Embodiment 4

A formula is as follows: 24 g of liquid rubber with a molecular weight ranging from 2000 to 5000, 12g of mineral oil, 20 g of plant polysaccharide, 1g of Lithospermum Root, 1 g of Angelica sinensis and 3g of sesame oil.

The Lithospermum Root, the Angelica sinensis and the sesame oil are accurately weighed according to the formula; the sesame oil is heated to 90°C and then the weighed Lithospermum Root and the Angelica sinensis are added, decocted for 1h, and filtered to obtain medicinal oil; and the obtained medicinal oil, the liquid rubber with the molecular weight ranging from 2000 to 5000, the mineral oil and the plant polysaccharide are evenly mixed in a mixed ratio, then put into and stirred in a double planetary stirrer, and then a mixed paste is introduced into an automatic filling machine to finish filling.

The formula and preparation steps are the same as those of Embodiment 2, except that iota-carrageenan and sericite powder are not added in the formula.

### Experiment 1. Sample material performance test

Paste products finally obtained in above Embodiments 1-3 and Comparative Embodiments 1-4 were tested respectively.

A certain amount (10g) of the paste is squeezed into a 100ml beaker, a total weight M1 of the paste and the beaker is accurately weighed; 50ml of artificial intestinal fluid is taken into the beaker containing the paste, the beaker containing the paste and the artificial intestinal fluid is placed in a constant-temperature incubator at 37°C, sealed and stored for 3 days; then solution in the beaker is carefully filtered, remaining paste and the beaker are placed in an oven at 60°C for 18h and taken out, a weight M2 at this time is weighed, and a corrosion resistance rate of the paste is {1-(M1-M2)/10}^{∗}100%; The above test was repeated for 3 times, and an average value was recorded as corrosion resistance of the paste.

Results of material performance test are shown in Table 1:

**Table 1**

| Items | Corrosion Resistance Rate |
|---|---|
| Embodiment 1 | 91% |
| Embodiment 2 | 93% |
| Embodiment 3 | 94% |
| Comparative Embodiment 1 | 56% |
| Comparative Embodiment 2 | 76% |
| Comparative Embodiment 3 | 94% |
| Comparative Embodiment 4 | 9% |

Results are analyzed as follows. In Embodiments 1-3 and Comparative Embodiment 3, due to addition of a certain amount of the iota-carrageenan and the sericite powder, test results of the paste's corrosion resistant rate show that its corrosion resistance rate is more than 90%, which indicates effect of corrosion resistance and no ease of disintegration in the intestinal fluid. There is no iota-carrageenan in Comparative Embodiment 1, and the paste is easy to disintegrate when heated, with a low corrosion resistance rate; In Comparative Example 2, there was no sericite powder, and without synergistic effect with iota-carrageenan, the paste partially disintegrated, with a relatively low corrosion resistance. In Comparative Example 4, there was no iota-carrageenan and sericite powder, and without carrageenan and sericite powder, the paste would all be disintegrated by the intestinal fluid, and an original shape cannot be maintained, with a poor corrosion resistance rate.

### Experiment 2: Clinical Trial Results

A total of 125 patients with faecal dermatitis who insisted on using the paste according to the present disclosure were recruited. In a first group, 29 cases were treated with the leak-proof paste prepared in the formula of the present disclosure in Embodiment 1; In a second group, 28 cases were treated with the leak-proof paste prepared in the formula of Embodiment 2; In a third group, 28 cases were treated with the leak-proof paste prepared in the formula of Embodiment 3; In a fourth group, 24 cases were treated with the leak-proof paste prepared in the formula of Comparative Example 3; and in a fifth group, 16 cases were treated with the leak-proof paste prepared in the formula of Comparative Example 4.

### One month after the test:

Cases with the paste disintegrated within 3 days: 1 case in the first group; 2 cases in the second group; 1 case in the third group; 1 case in the fourth group; 13 cases in the fifth group, with a total effective rate of 95.3%.

Case without alleviated inflammation of fecal dermatitis: 0 case in the first group; 1 case in the second group; 0 case in the third group; 18 cases in the fourth group; 14 cases in the fifth group, with a total effective rate of 98.8%.

Causes of main differences are analyzed as follows.

The iota-carrageenan can form a strong gel network structure with calcium ions in the stoma excreta. The gel network structure is elastic and insoluble in water and its strength increases with increase of the calcium ions in solution, so the paste can self-regulate its strength with amount of the stoma excreta. In addition, the sericite has unique volume expansion effect and a two-dimensional sheet configuration with a high aspect ratio, excellent reinforcement effect is exhibited in rubber products, so it can strengthen the paste jointly with the iota-carrageenan. In addition, the sericite powder presents acid and alkali corrosion resistance, and the iota-carrageenan and the sericite powder cooperate to achieve high paste strength and good corrosion resistance. According to results of Embodiments 1-3 and Comparative Embodiments 1-2 and 4, the iota-carrageenan and the sericite have synergistic effect on the corrosion resistance.

The Lithospermum Root contains shikonin, which has good effect of eliminating putridity and engendering flesh. At the same time, the Lithospermum Root can inhibit growth activity of Candida albicans, and protect and promote healing of wounds at the same time. The Angelica sinensis can promote blood circulation, nourish blood and promote granulation. The sesame oil has efficacy of detoxifying and promoting granulation. It can be seen from inflammatory situations of fecal dermatitis of users in Embodiments 1-3 and Comparative Embodiment 3 that Combination of these three medicines presents remarkable astringent effect in treating skin diseases, so it can achieve astringent effect for skin complications and prevent sores.

## Claims

1. A corrosion-resistant and leak-proof stoma paste with self-regulation of strength, wherein the paste's formula comprises 4-40 parts of liquid rubber with a molecular weight ranging from 2000 to 5000, 8-18 parts of mineral oil, 14-34 parts of iota-carrageenan, 10-20 parts of plant polysaccharide and 15-20 parts of sericite, by weight.

2. The corrosion-resistant and leak-proof stoma paste with self-regulation of strength according to claim 1, wherein the formula further comprises 5-15 parts of the stoma skin complication medicine by weight.

3. The corrosion-resistant and leak-proof stoma paste with self-regulation of strength according to claim 2, wherein the stoma skin complication medicine comprises 1-3 parts of Lithospermum Root, 1-3 parts of Angelica sinensis and 3-9 parts of sesame oil, by weight.

4. The corrosion-resistant and leak-proof stoma paste with self-regulation of strength according to claim 3, wherein a weight ratio of the Lithospermum Root to the sesame oil is 1: 3.

5. The corrosion-resistant and leak-proof stoma paste with self-regulation of strength according to claim 1, wherein the liquid rubber with the molecular weight ranging from 2000 to 5000 is one or more of polyisobutylene, liquid polyisoprene and liquid styrene-butadiene rubber with a molecular weight ranging from 2000 to 5000.

6. The corrosion-resistant and leak-proof stoma paste with self-regulation of strength according to claim 1, wherein the mineral oil is one or more of naphthenic oil, white oil and paraffin oil.

7. The corrosion-resistant and leak-proof stoma paste with self-regulation of strength according to claim 1, wherein the plant polysaccharide is one or more of tamarind seed polysaccharide, seaweed polysaccharide, chitosan oligosaccharide, tremella polysaccharide, hawthorn polysaccharide and oat β - glucan.

8. A preparation method of a corrosion-resistant and leak-proof stoma paste with self-regulation of strength, comprising:
Step 1, preparing materials according to a formula;
the formula comprising 4-40 parts of liquid rubber with a molecular weight ranging from 2000 to 5000, 8-18 parts of mineral oil, 14-34 parts of iota-carrageenan, 10-20 parts of plant polysaccharide and 15-20 parts of sericite, by weight; and
Step 2, evenly mixing the liquid rubber with the molecular weight ranging from 2000 to 5000, the mineral oil, the iota-carrageenan, the plant polysaccharide and the sericite, and stirring to obtain a mixed paste, which is packaged to obtain a finished product.

9. A preparation method of a corrosion-resistant and leak-proof stoma paste with self-regulation of strength, comprising:
Step 1, preparing materials according to a formula;
the formula comprising 4-40 parts of liquid rubber with a molecular weight ranging from 2000 to 5000, 8-18 parts of mineral oil, 14-34 parts of iota-carrageenan, 10-20 parts of plant polysaccharide and 15-20 parts of sericite, 1-3 parts of Lithospermum Root, 1-3 parts of Angelica sinensis and 3-9 parts of sesame oil, by weight;
Step 2, heating the sesame oil to 90°C and then adding the weighed Lithospermum Root and the Angelica sinensis, decocting for 1h, and filtering to obtain medicinal oil; and
Step 3, evenly mixing the obtained medicinal oil, the liquid rubber with the molecular weight ranging from 2000 to 5000, the mineral oil, the iota-carrageenan, the plant polysaccharide and the sericite, and stirring to obtain a mixed paste, which is packaged to obtain a finished product.

10. The preparation method of the corrosion-resistant and leak-proof stoma paste with self-regulation of strength according to claim 8 or 9, wherein a double planetary stirrer is adopted for stirring, with a rotating speed of 80-180 r/min.

## Patentansprüche

1. Korrosionsbeständige und lecksichere Stomapaste mit Selbstregulierung der Stärke, wobei die Formel der Paste 4-40 Teile Flüssigkautschuk mit einer Molmasse, die von 2000 bis 5000 reicht, 8-18 Teile Mineralöl, 14-34 Teile Iota-Carrageen, 10-20 Teile pflanzliches Polysaccharid und 15-20 Teile Serizit, bezogen auf das Gewicht, umfasst.

2. Korrosionsbeständige und lecksichere Stomapaste mit Selbstregulierung der Stärke nach Anspruch 1, wobei die Formel weiterhin 5-15 Teile der Stoma-Hautkomplikationsmedizin, bezogen auf das Gewicht, umfasst.

3. Korrosionsbeständige und lecksichere Stomapaste mit Selbstregulierung der Stärke nach Anspruch 2, wobei die Stoma-Hautkomplikationsmedizin 1-3 Teile Lithospermum-Wurzel, 1-3 Teile Angelica sinensis und 3-9 Teile Sesamöl, bezogen auf das Gewicht, umfasst.

4. Korrosionsbeständige und lecksichere Stomapaste mit Selbstregulierung der Stärke nach Anspruch 3, wobei ein Gewichtsverhältnis der Lithospermum-Wurzel zu dem Sesamöl 1:3 beträgt.

5. Korrosionsbeständige und lecksichere Stomapaste mit Selbstregulierung der Stärke nach Anspruch 1, wobei der Flüssigkautschuk mit der Molmasse, die von 2000 bis 5000 reicht, eines bzw. einer oder mehrere von Polyisobutylen, flüssigem Polyisopren und flüssigem Styrol-Butadien-Kautschuk mit einer Molmasse, die von 2000 bis 5000 reicht, ist.

6. Korrosionsbeständige und lecksichere Stomapaste mit Selbstregulierung der Stärke nach Anspruch 1, wobei das Mineralöl eines oder mehrere von naphthenischem Öl, Weißöl und Paraffinöl ist.

7. Korrosionsbeständige und lecksichere Stomapaste mit Selbstregulierung der Stärke nach Anspruch 1, wobei das pflanzliche Polysaccharid eines oder mehrere von Tamarindensamen-Polysaccharid, Seetang-Polysaccharid, Chitosan-Oligosacharid, Tremella-Polysaccharid, Weißdorn-Polysaccharid und Hafer-β-Glucan ist.

8. Herstellungsverfahren einer korrosionsbeständigen und lecksicheren Stomapaste mit Selbstregulierung der Stärke, umfassend:
Schritt 1, Herstellen von Materialien gemäß einer Formel;
wobei die Formel 4-40 Teile Flüssigkautschuk mit einer Molmasse, die von 2000 bis 5000 reicht, 8-18 Teile Mineralöl, 14-34 Teile Iota-Carrageen, 10-20 Teile pflanzliches Polysaccharid und 15-20 Teile Serizit, bezogen auf das Gewicht, umfasst; und
Schritt 2, gleichmäßiges Mischen des Flüssigkautschuks mit der Molmasse, die von 2000 bis 5000 reicht, des Mineralöls, des Iota-Carrageens, des pflanzlichen Polysaccharids und des Serizits und Rühren, um eine gemischte Paste zu erhalten, die verpackt wird, um ein Endprodukt zu erhalten.

9. Herstellungsverfahren einer korrosionsbeständigen und lecksicheren Stomapaste mit Selbstregulierung der Stärke, umfassend:
Schritt 1, Herstellen von Materialien gemäß einer Formel;
wobei die Formel 4-40 Teile Flüssigkautschuk mit einer Molmasse, die von 2000 bis 5000 reicht, 8-18 Teile Mineralöl, 14-34 Teile Iota-Carrageen, 10-20 Teile pflanzliches Polysaccharid und 15-20 Teile Serizit, 1-3 Teile Lithospermum-Wurzel, 1-3 Teile Angelica sinensis und 3-9 Teile Sesamöl, bezogen auf das Gewicht, umfasst;
Schritt 2, Erhitzen des Sesamöls auf 90 °C und dann Zugeben der gewogenen Lithospermum-Wurzel und der Angelica sinensis, Abkochen für 1 h und Filtrieren, um medizinisches Öl zu erhalten; und
Schritt 3, gleichmäßiges Mischen des erhaltenen medizinischen Öls, des Flüssigkautschuks mit der Molmasse, die von 2000 bis 5000 reicht, des Mineralöls, des Iota-Carrageens, des pflanzlichen Polysaccharids und des Serizits und Rühren, um eine gemischte Paste zu erhalten, die verpackt wird, um ein Endprodukt zu erhalten.

10. Herstellungsverfahren einer korrosionsbeständigen und lecksicheren Stomapaste mit Selbstregulierung der Stärke nach Anspruch 8 oder 9, wobei ein Doppelplanetenrührwerk zum Rühren mit einer Drehzahl von 80-180 U/min eingerichtet ist.

## Revendications

1. Pâte de stomie résistante à la corrosion et étanche avec autorégulation de la résistance, dans laquelle la formule de la pâte comprend entre 4 et 40 parties de caoutchouc liquide d'un poids moléculaire allant de 2 000 à 5 000, entre 8 et 18 parties d'huile minérale, entre 14 à 34 parties d'iota-carraghénane, entre 10 et 20 parties de polysaccharide végétal et entre 15 et 20 parties de séricite, en poids.

2. Pâte de stomie résistante à la corrosion et étanche avec autorégulation de la résistance selon la revendication 1, dans laquelle la formule comprend en outre entre 5 et 15 parties du médicament de complication de la peau de la stomie en poids.

3. Pâte de stomie résistante à la corrosion et étanche avec autorégulation de la résistance selon la revendication 2, dans laquelle le médicament de complication de la peau de stomie comprend entre 1 et 3 parties de racine de Lithospermum, entre 1 et 3 parties d'Angelica sinensis et entre 3 et 9 parties d'huile de sésame, en poids.

4. Pâte de stomie résistante à la corrosion et étanche avec autorégulation de la résistance selon la revendication 3, dans laquelle un rapport de poids de la racine de Lithospermum à l'huile de sésame est de 1: 3.

5. Pâte de stomie résistante à la corrosion et étanche avec autorégulation de la résistance selon la revendication 1, dans laquelle le caoutchouc liquide de poids moléculaire allant de 2 000 à 5 000 est un ou plusieurs parmi du polyisobutylène, du polyisoprène liquide et du caoutchouc styrène-butadiène liquide dont le poids moléculaire varie de 2 000 à 5 000.

6. Pâte de stomie résistante à la corrosion et étanche avec autorégulation de la résistance selon la revendication 1, dans laquelle l'huile minérale est une ou plusieurs parmi de l'huile naphténique, de l'huile blanche et de l'huile de paraffine.

7. Pâte de stomie résistante à la corrosion et étanche avec autorégulation de la résistance selon la revendication 1, dans laquelle le polysaccharide végétal est un ou plusieurs parmi un polysaccharide de graines de tamarin, un polysaccharide d'algues, un oligosaccharide de chitosane, un polysaccharide de tremella, un polysaccharide d'aubépine et le β-glucane d'avoine.

8. Procédé de préparation d'une pâte de stomie résistante à la corrosion et étanche avec autorégulation de la résistance, comprenant :
Étape 1, la préparation des matériaux selon une formule ;
la formule de la pâte comprenant entre 4 et 40 parties de caoutchouc liquide d'un poids moléculaire allant de 2 000 à 5 000, entre 8 et 18 parties d'huile minérale, entre 14 et 34 parties d'iota-carraghénane, entre 10 et 20 parties de polysaccharide végétal et entre 15 et 20 parties de séricite, en poids ; et
Étape 2, le mélange uniforme du caoutchouc liquide de poids moléculaire allant de 2 000 à 5 000, l'huile minérale, l'iota-carraghénane, le polysaccharide végétal et la séricite, et l'agitation pour obtenir une pâte mélangée, qui est emballée pour obtenir un produit fini.

9. Procédé de préparation d'une pâte de stomie résistante à la corrosion et étanche avec autorégulation de la résistance, comprenant :
Étape 1, la préparation des matériaux selon une formule ;
la formule de la pâte comprenant entre 4 et 40 parties de caoutchouc liquide d'un poids moléculaire allant de 2 000 à 5 000, entre 8 et 18 parties d'huile minérale, entre 14 à 34 parties d'iota-carraghénane, entre 10 et 20 parties de polysaccharide végétal et entre 15 et 20 parties de séricite, entre 1 et 3 parties de racine de Lithospermum, entre 1 et 3 parties d'Angelica sinensis et entre 3 et 9 parties d'huile de sésame, en poids ;
Etape 2, le chauffage de l'huile de sésame à 90 °C, puis l'ajout de la racine de Lithospermum et de l'Angelica sinensis pesées, la décoction pendant 1 h et la filtration pour obtenir de l'huile médicinale ; et
Étape 3, le mélange uniforme de l'huile médicinale obtenue, le caoutchouc liquide de poids moléculaire allant de 2 000 à 5 000, l'huile minérale, l'iotacarraghénane, le polysaccharide végétal et la séricite, et l'agitation pour obtenir une pâte mélangée, qui est emballée pour obtenir un produit fini.

10. Procédé de préparation de la pâte de stomie résistante à la corrosion et étanche avec autorégulation de la résistance selon la revendication 8 ou 9, dans lequel un agitateur planétaire double est adopté pour l'agitation, avec une vitesse de rotation entre 80 et 180 r/min.
